# EUROPEAN PATENT SPECIFICATION

(11) **EP 1 593 366 B1**
(45) Date of publication and mention of the grant of the patent: **31.12.2008**
(21) Application number: 05008543.0
(22) Date of filing: 19.04.2005
(51) Int. Cl.: A61K 8/73, A61K 8/92, A61Q 1/00, A61Q 1/12

(54) **Cosmetic pressed powder**
Kosmetischer komprimierter Puder
Poudre compacte cosmetique

(30) Priority: 20.04.2004 JP 2004124114; 10.12.2004 JP 2004357972
(43) Date of publication of application: 09.11.2005
(73) Proprietor: KAO CORPORATION, Tokyo 103-8210 (JP)
(72) Inventor: Imai, Takamitsu, Kao Corp.Research Laboratories, Tokyo 131-8501 (JP)
(74) Representative: HOFFMANN EITLE

(56) References cited:
- EP-A- 1 352 625
- US-B1- 6 342 239
- US-B1- 6 482 441
- PATENT ABSTRACTS OF JAPAN vol. 2003, no. 08, 6 August 2003 (2003-08-06) & JP 2003 095846 A (KOSE CORP), 3 April 2003 (2003-04-03)
- PATENT ABSTRACTS OF JAPAN vol. 2002, no. 11, 6 November 2002 (2002-11-06) & JP 2002 193749 A (KOSE CORP), 10 July 2002 (2002-07-10)

## Description

### BACKGROUND OF THE INVENTION

### Field of the Invention

The present invention relates to a cosmetic pressed powder.

### Background of the Invention

Cosmetic pressed powder is produced by mixing oil or the like into a powder and then milling and compacting the mixture. Some studies have been conducted to improve its qualities in use, such as attachment thereof to an applicator or spreadability of the cosmetic when it is applied.

For example, JP-A-2003-95847 describes a cosmetic pressed powder, which contains a metallic soap and a dextrin fatty acid ester and is excellent in terms of quality in use and stability. JP-A-2000-355530 describes a cosmetic, which contains wheat bran and a dextrin fatty acid ester and is excellent in terms of attachment to the skin, spreadability, and safety. Moreover, JP-A-2004-277366 describes a cosmetic pressed powder containing liquid hydrocarbon oil and a dextrin fatty acid ester, which is obtained by filling a mixture containing a solvent and then removing the solvent.
JP 2003095846 discloses an oily solid cosmetic which includes a wax, an polar oil, a dextrin fatty acid ester, fine particles and further polybutene.
US 6,342,239 B1 discloses a powder dispersion in oil compositions comprising powder, oil and a copolymer, wherein said powder is dispersed in said oil, which oil contains said copolymer composed of constituent units from a monomer (A) and constituent units from a monomer (B).
EP 1 352 625 A1 discloses a cosmetic composition comprising (a) composite particles obtainable by allowing at least two kinds of particles having different shapes and particle diameters to contact at least one kind of polymer compounds selected from fluorinated polymer compounds, silicone polymer compounds, and mixtures thereof in the presence of supercritical carbon dioxide, and (b) cosmetic components.
US 6,482,441 B1 discloses a surface-treated powder, comprising a powder usable for cosmetics and the coating layers of surface treating agents of a layer A and a layer B, each formed on at least a portion of the powder particle surface. Layer A is a coating layer of a surface treating agent that is a solid at room temperature. Layer B is a coating layer of a surface treating agent that is a liquid at room temperature. However, such improvement of the quality in use of a cosmetic leads to problems such as a lowered strength of the final product or lowered stabilities, including shape retention, over time. Thus, it has been difficult to satisfy the requirements of both utility and stability.

### SUMMARY OF THE INVENTION

The present invention provides a cosmetic pressed powder containing a wax (A), a dextrin fatty acid ester (B), a liquid oil (C), and a powder (D), wherein the content of the component (D) is 80% by weight or more.

### DETAILED DESCRIPTION OF THE PREFERRED EMBODIMENTS

The present invention relates to a cosmetic pressed powder having excellent quality in use and stability.

The present inventors have found that when a wax and a dextrin fatty acid ester are uniformly dispersed in a powder, it is possible to obtain a cosmetic pressed powder excellent in terms of attachment to an applicator, spreadability when it is applied, conformability, and quality in use, as well as shape retention and impact resistance.

The cosmetic pressed powder of the present invention is excellent in terms of quality in use and stability, and it can be finely and uniformly attached to an applicator. When this cosmetic pressed powder is applied, it is excellent in terms of spreadability and conformability, providing a finely textured finish. In addition, this cosmetic pressed powder is excellent in terms of impact resistance and shape retention when it is transported or dropped accidentally. Moreover, it lasts long on the skin.

The wax as component (A) used in the present invention is in a solid state at 25°C, and reversibly changes between solid and liquid states. It is a hydrophobic compound having a melting point of 40°C or higher, and preferably 55°C or higher. The wax as component (A) may be provided as a wax consisting of components such as a hydrocarbon, an ester, and a silicone. Examples of such a wax may include an animal wax, a plant wax, a mineral wax, a synthetic wax, and a mixture thereof. Specific examples of such a wax may include: animal waxes such as beeswax or whale wax; plant waxes such as carnauba wax, candelilla wax, rice wax, or Japan wax; mineral waxes such as montan wax, ozokerite, ceresin, paraffin wax, petrolatum, or microcrystalline wax; and synthetic waxes such as polyethylene wax, Fisher-Tropsch wax, hydrogenated castor oil, hydrogenated jojoba oil, 12-hydroxystearic acid, amide stearate, imide phthalic anhydride, or silicone wax. Of these, microcrystalline wax and beeswax are preferable for their excellent impact resistance.

Among the aforementioned waxes, those with a penetration number measured by the method described in ASTM D-1321 of between 20 and 110 at 25°C are preferable because of their higher impact resistance. Waxes containing a microcrystalline wax and having a penetration number of 20 to 110 at 25°C are preferable because they exhibit high impact resistance and also allow for fine attachment of cosmetics to an applicator.
Regarding wax, a wax having a penetration number between 20 and 110 and other waxes may be used in combination. When several types of waxes are used in combination, it is preferable that a wax having a penetration number between 20 and 110 be contained at a ratio of 60% by weight or more, based on the total weight of the waxes.

The wax as component (A) may contain more than one waxes. It is preferable that 0.1% to 5% by weight, and more preferably 1% to 4% by weight of the wax as component (A) is contained in the total composition of a cosmetic pressed powder in view of impact resistance and fine attachment of cosmetics to an applicator.

As a dextrin fatty acid ester as component (B) used in the present invention, an ester compound having a fatty acid containing 8 to 24 carbon atoms and dextrin having a average polymerization degree of 3 to 150 is preferable. Specific examples may include dextrin palmitate, dextrin stearate, palmitic acid-dextrin stearate, dextrin oleate, dextrin isopalmitate, dextrin isostearate, dextrin myristate, and palmitic acid-dextrin 2-ethylhexanoate. Of these, dextrin palmitate, dextrin myristate, and palmitic acid-dextrin 2-ethylhexanoate are preferable in terms of the long-lasting property on the skin cosmetics from those. Of these, dextrin palmitate is more preferable.

One or more types of dextrin fatty acid esters may be used in combination as component (B). It is preferable that 0.01% to 10% by weight, preferably 0.1% to 4% by weight, and more preferably 0.2% to 2% by weight of the component (B) be contained in the total composition in view of impact resistance on the product level and fine attachment of resulting cosmetics to an applicator.

A liquid oil as component (C) used in the present invention is not limited as long as it is used for common cosmetics. It is preferable to use a liquid oil, in which the wax as component (A) and the dextrin fatty acid ester as component (B) can be dissolved. Examples of such a liquid oil may include: oils and fats such as cacao butter, castor oil, jojoba oil, olive oil, sunflower oil, or macadamia nut oil; higher fatty acids such as isononanoic acid or isostearic acid; fatty acid esters such as isopropyl myristate, isopropyl isostearate, neopentylglycol dicaprate, or diisostearyl malate; hydrocarbon oils such as liquid paraffin, liquid isoparaffin, or squalane; silicone oils; and fluorine oils. Of these, isopropyl isostearate, liquid isoparaffin, squalane, and neopentylglycol dicaprate are preferable in view of the long-lasting property of resulting cosmetics on the skin. Of these, isopropyl isosterate and liquid isoparaffin are preferable.

A liquid oil having a viscosity at 25°C of 1,000 mPa·s or less, and more preferably 1 to 200 mPa·s, is preferable in view of fine attachment of resulting cosmetics to an applicator and a finely textured finish. When two or more types of liquid oils are used, the viscosity of a liquid oil of the highest content is preferably 1,000 mPa·s or less at 25°C, and more preferably 200 mPa·s or less. Such a viscosity is measured at 25°C at a number of rotations of 6 rpm using a rotational viscometer (B8L, Toki Sangyo Co., Ltd.). A rotor is appropriately selected from Nos. 1 to 4.

One or more types of liquid oils may be used as the liquid oil as component (C). It is preferable that 1% to 15% by weight, and more preferably 3% to 10% by weight of the liquid oil as component (C) be contained in the total composition in terms of spreadability of resulting cosmetics when they are applied, and high conformability thereof to the skin.

A powder as component (D) used in the present invention is not limited as long as it is used for common cosmetics. A powder has an average particle size of preferably between 0.001 and 200 µm. Examples of a powder may include: an inorganic powder such as silicic acid anhydride, magnesium silicate, talc, sericite, mica, kaolin, iron red, clay, bentonite, bismuth oxychloride, zirconium oxide, magnesium oxide, zinc oxide, aluminum oxide, calcium sulfate, barium sulfate, magnesium sulfate, calcium carbonate, magnesium carbonate, iron oxide, ultramarine blue, chromium oxide, chromium hydroxide, carmine, carbon black, pearlescent pigment, or a complex thereof; an organic powder such as polyamide, polyester, polypropylene, polystyrene, polyurethane, vinyl resin, urea resin, phenol resin, silicone resin, acrylic resin, melamine resin, epoxy resin, polycarbonate resin, divinylbenzene-styrene copolymer, silk powder, cellulose, CI pigment yellow, CI pigment orange, metal salts of long-chain alkyl phosphate, metal salts of higher fatty acids, N-mono long-chain alkyl acyl basic amino acid, and a complex thereof; and complexes of the aforementioned an inorganic powder and an organic powder.

Of these, as a pearlescent pigment a pearlescent pigment that is a coloring agent containing a thin base material and a coating layer, wherein the base material is selected from the group consisting of mica, synthetic mica, silica, and glass, and the coating layer is selected from the group consisting of metal, metal oxide, metallic complex, and organic pigment, may be used. Such a pearlescent pigment has an average particle size preferably of 10 µm or greater, and more preferably between 10 and 100 µm. In terms of fine attachment of resulting cosmetics to an applicator and a finely textured finish, the pearlescent pigment is contained in the total composition at a ratio of preferably 20% by weight or more, and more preferably 20% to 60% by weight.
An average particle size of the powder herein is a value (median size) obtained by use of an apparatus for measuring particle size distribution using laser diffraction (product of Horiba Ltd., LA-920)

The powder as component (D) may also be subjected to surface treatment before use. Examples of such a surface treatment may include a silicone treatment, a fatty acid treatment, an amino acid treatment, a lecithin treatment, a metal soap treatment, an alkyl treatment, a fluorine compound treatment, an ester treatment, and a combined use of these treatments.
Specifically, such a silicone treatment may be a treatment using methyl hydrogen polysiloxane, methyl polysiloxane, trimethyl siloxysilicate, silicone resin, or the like; such a fatty acid treatment may be a treatment using myristic acid, stearic acid, or the like; and such a fluorine compound treatment may be a treatment using perfluoroalkyl phosphate, perfluoroalkylsilane, or the like.

The amount of a surface-treating agent used is preferably 0.1 to 10 parts by weight, and more preferably 2 to 9 parts by weight with respect to the weight of a powder before the treatment. A method for making powder surface hydrophobic is not limited, and it can be carried out according to ordinary methods.

The powder as component (D) is contained in the total composition at a ratio of 80% by weight or more, preferably 85% to 96% by weight, and more preferably 88% to 95% by weight.

In the present invention, in view of a finely textured finish and shape retention of the product, the weight ratio (B)/(A) of the wax as component (A) and the dextrin fatty acid ester as component (B) is preferably 0.1 to 0.5, and more preferably 0.2 to 0.4. In addition, in terms of the long-lasting property of resulting cosmetics on the skin, the weight ratio ((A)+(B))/(C) of the total weight of the wax as component (A) and the dextrin fatty acid ester as component (B), and the liquid oil as component (C), is preferably 0.14 to 1.1, and more preferably 0.35 to 0.64.

Other than the aforementioned components, the cosmetic pressed powder of the present invention may appropriately comprise components that are commonly used for cosmetics, such as a surfactant, an antiseptic, an antioxidant, a pigment, a perfume, an ultraviolet absorber, a moisturizer, a bactericide, or a skin activator.

The cosmetic pressed powder of the present invention can be produced, for example, by heating and dissolving a wax (A) and a dextrin fatty acid ester (B) in a liquid oil (C), then adding a powder (D) thereto, and compacting the obtained mixture. It is preferable to produce the cosmetic pressed powder of the present invention by uniformly mixing a wax (A), a dextrin fatty acid ester (B), a liquid oil (C), and a powder (D), at a temperature where the wax (A) and the dextrin fatty acid ester (B) can be dissolved in the liquid oil (C). Specifically, it is preferable that an oil component obtained by dissolving the wax (A) and the dextrin fatty acid ester (B) in the liquid oil (C) with heat be added to the powder (D), which has previously been blended at the temperature where (A) and (B) could be dissolved in (C), so that the components are uniformly mixed. In order to add the oil component, it is also possible to spray it using a spray. The obtained mixture can be directly compacted. However, it is preferable that the mixture is cooled to room temperature and then milled, followed by compaction.

The cosmetic pressed powder of the present invention is preferably used as makeup cosmetics such as foundation, face powder, solid face powder, eye shadow, eyebrow, or rouge, or as body powder.

### EXAMPLES

The following evaluation methods were applied in the examples.

### (Evaluation method)

### (1) Measurement of penetration number:

A wax component was melted with heat and then cooled. The obtained product was used as a sample. The penetration number of the sample was measured using a Testing Apparatus for Penetration (Nikka Engineering).

The sample was heated to a temperature that was 17°C higher than the melting point thereof, so that it was melted. Two cork stoppers (No. 16) were placed in line on a horizontal stand, and a brass plate was placed thereon. On the top face thereof, a parting agent (which was obtained by mixing glycerin and water at equivalent amounts) was thinly applied. A predetermined sample vessel (a brass cylinder with an inside diameter of 25.4 mm, a height of 31.8 mm, and a wall thickness of 3.2 mm) was placed on the brass plate. Thereafter, a melted sample was poured into the sample vessel to such an extent that it could be seen from the superior margin of the vessel in a mound shape, and it was stood to cool at room temperature (22°C to 26°C) for 1 hour. Thereafter, the vessel was removed from the brass plate and then left in water with a constant temperature of 25°C for 1 hour. A predetermined needle having a total weight of 100 g was vertically penetrated into the sample for 5 seconds, and the dial gauge was read at the time, to measure the depth of the penetrated needle. The penetration number of the sample was indicated with a value obtained by measuring the depth of the penetrated needle at a unit of 0.1 mm and then increasing the value 10 times. Four measurement values were averaged, and the first decimal point of the average value was rounded. The resulting value was defined as a penetration number.

### (2) Convenience:

Ten special panelists used various cosmetic pressed powder to conduct a sensory evaluation of the cosmetic pressed powder in terms of fine attachment of the cosmetics to an applicator, spreadability of the cosmetics when they were applied, conformability thereof, a finely textured finish, and a long-lasting property. These factors were evaluated in accordance with the following standards.
E: 7 or more panelists evaluated the cosmetic positively.
G: 4 to 6 panelists evaluated the cosmetic positively.
M: 2 or 3 panelists evaluated the cosmetic positively.
P: 1 panelist or none evaluated the cosmetic positively.

### (3) Impact resistance:

A middle plate with a diameter of 2.5 cm that had been filled with cosmetic pressed powder followed by compaction was placed in a vessel. Thereafter, from a height of 50 cm, the vessel was repeatedly dropped onto a lauan blockboard with a thickness of 25 mm. Based on the number of dropping that was necessary for the occurrence of abnormality such as a chip or crack, impact resistance was evaluated in accordance with the following standards.
E: 15 times or more
G: 10 to 14 times
M: 5 to 9 times
P: 1 to 4 times

### Examples 1 to 9 and Comparative examples 1 to 3

Cosmetic pressed powder with the compositions shown in Table 1 were produced. The produced cosmetics were used and evaluated in terms of fine attachment of the cosmetics to an applicator, spreadability of the cosmetics when they were applied, conformability thereof, a finely textured finish, a long-lasting property, and impact resistance. The results are also shown in Table 1.

### (Production method)

Powdery components were mixed and then heated to 80°C to 90°C. Separately, a mixture of a wax component, a dextrin fatty acid ester component and a liquid oil component was heated to 80°C to 90°C to melt it. The thus melted product was then added to the above mixture and was uniformly blended. The resulting mixture was cooled and then milled. The resultant product was filled in a middle plate, followed by compaction, so as to obtain a cosmetic pressed powder.

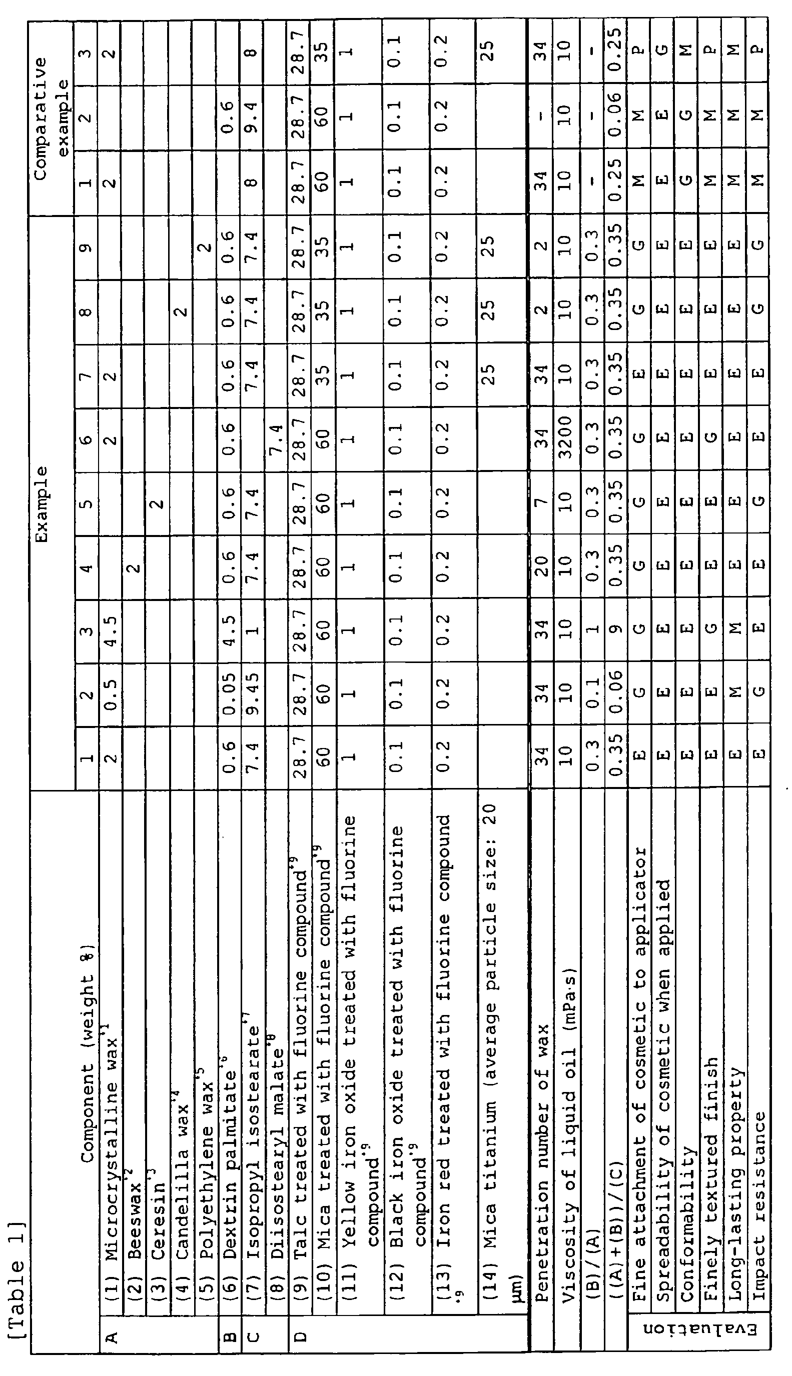

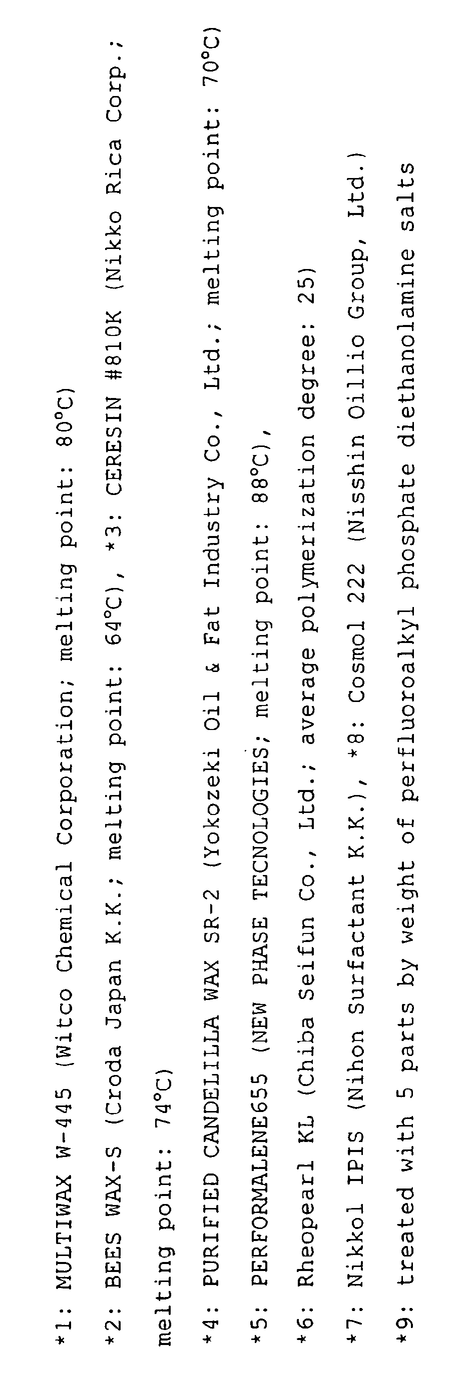

### Examples 10 to 16

Cosmetic pressed powder with the compositions shown in Table 2 were produced in the same manner as in Examples 1 to 9. The produced cosmetics were evaluated in terms of fine attachment of the cosmetics to an applicator, spreadability of the cosmetics when they were applied, conformability thereof, a finely textured finish, a long-lasting property, and impact resistance. The results are also shown in Table 2.

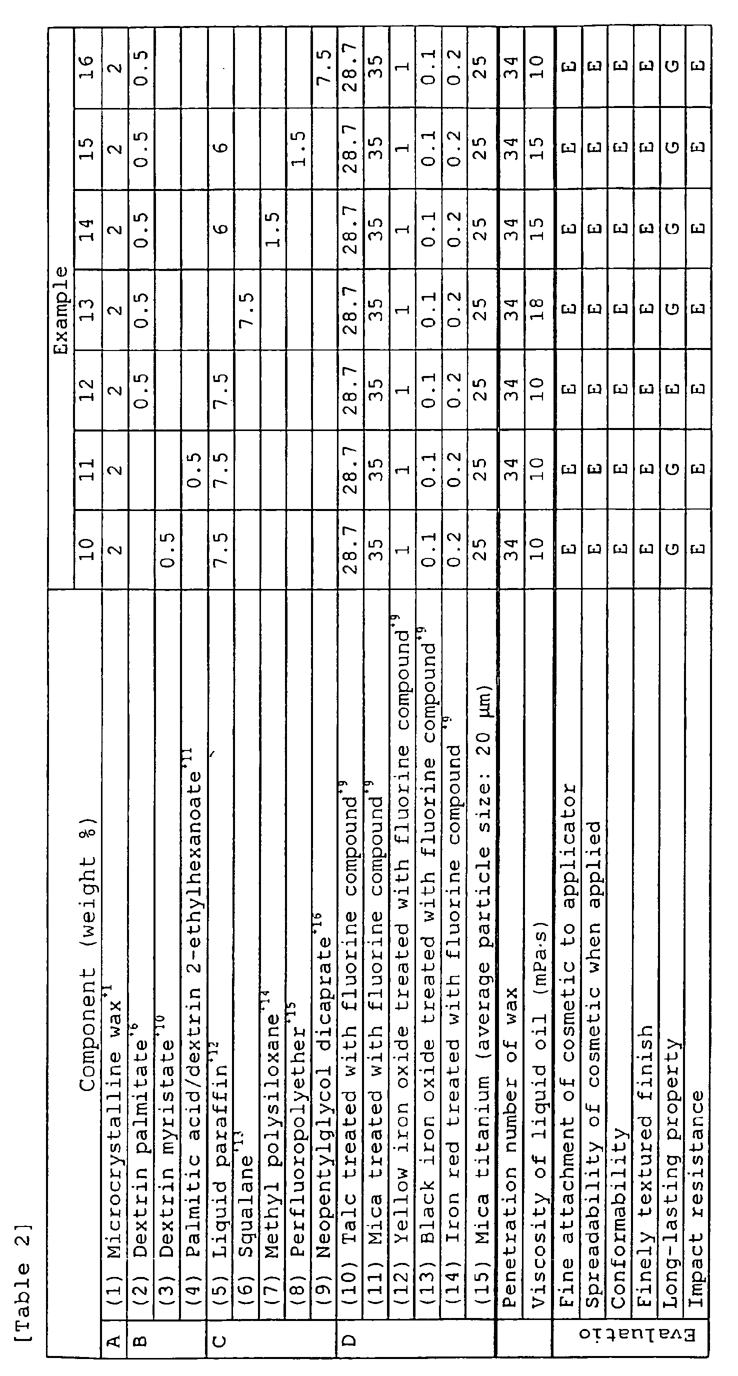

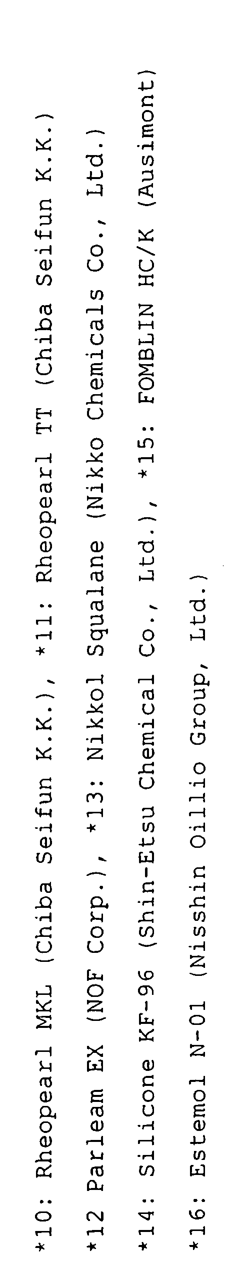

### Example 17 (Eye shadow)

An eye shadow with the composition shown in Table 3 was produced.

### (Production method)

Components (1) to (10) were mixed with components (14) and (15), and the mixture was then heated to 80°C to 90°C. Components (11) to (13) were heated to 80°C to 90°C to melt them together, and these components were mixed. The resulting mixture was added to the above mixture and was uniformly blended. The resulting mixture was cooled and then milled. Thereafter, the resultant product was filled in a middle plate, followed by compaction, so as to obtain an eye shadow.

**[Table 3]**

| (Component) | (Weight %) |
|---|---|
| (1) Surface treated talc (average particle size: 6 µm)^{*17} | 24.1 |
| (2) Surface treated synthetic mica (average particle size: 10 µm)^{*17} | 29 |
| (3) Spherical nylon powder (average particle size: 5 µm) | 5 |
| (4) Titanium oxide | 0.1 |
| (5) Black iron oxide | 0.05 |
| (6) Ultramarine blue | 0.3 |
| (7) Red #226 | 0.3 |
| (8) Mica titanium (average particle size: 20 µm)^{*18} | 20 |
| (9) Mica titanium coated with iron red (average particle size: 20 µm)^{*19} | 8 |
| (10) Glass flake coated with titanium oxide (average particle size: 80 µm)^{*20} | 3 |
| (11) Microcrystalline wax (penetration number: 34)^{*1} | 2 |
| (12) Dextrin palmitate^{*6} | 0.5 |
| (13) Liquid isoparaffin (viscosity: 15mPa·s)^{*12} | 7.5 |
| (14) Antiseptic | 0.1 |
| (15) Perfume | 0.05 |

| | |
|---|---|
| *17: treated with 5 parts by weight of perfluoroalkyl phosphate diethanolamine salts and 2 parts by weight of n-octyl triethoxysilane *18: Flamenco Super Pearl (Engelhard Corp.) *19: Cloisonne Rouge Flambe (Engelhard Corp.) *20: Metashine MC1080RR (Nippon Sheet Glass Co., Ltd.) | |

### Example 18 (Eye shadow)

An eye shadow with the composition shown in Table 4 was produced.

### (Production method)

Components (1) to (14) were mixed with components (20) and (21), and the mixture was then heated to 80°C to 90°C. Components (15) to (19) were heated to 80°C to 90°C to melt them, and these components were mixed. The resulting mixture was added to the above mixture and was uniformly blended. The resulting mixture was cooled and then milled. Thereafter, the resultant product was filled in a middle plate, followed by compaction, so as to obtain an eye shadow.

**[Table 4]**

| (Component) | (Weight %) |
|---|---|
| (1) Surface treated talc (average particle size: 6 µm)^{*21} | 17.55 |
| (2) Surface treated mica (average particle size: 15 µm)^{*21} | 5 |
| (3) Surface treated sericite (average particle size: 8 µm)^{*21} | 12 |
| (4) Surface treated synthetic mica (average particle size: 10 µm)^{*21} | 10 |
| (5) Spherical silica (average particle size: 10 µm) | 2 |
| (6) Crosslinked silicone powder | 3 |
| (7) Surface treated titanium oxide^{*21} | 0.1 |
| (8) Surface treated yellow iron oxide^{*21} | 0.1 |
| (9) Surface treated iron red^{*21} | 0.1 |
| (10) Zinc stearate | 3 |
| (11) Synthetic mica coated with titanium oxide (average particle size: 100 µm)^{*22} | 5 |
| (12) Mica coated with titanium oxide/silica/titanium oxide (average particle size: 20 µm)^{*23} | 20 |
| (13) Silica flake coated with titanium oxide (average particle size: 20 µm)^{*24} | 10 |
| (14) PET/aluminum/epoxy laminated powder (average particle size: 150 µm) | 2 |
| (15) Microcrystalline wax (penetration number: 34)^{*1} | 2 |
| (16) Dextrin palmitate^{*6} | 0.5 |
| (17) Liquid isoparaffin (viscosity: 15mPa·s)^{*12} | 2.5 |
| (18) trimethyl siloxysilicate/methyl polysiloxane^{*25} | 2 |
| (19) Crosslinked silicone-methyl polysiloxane^{*26} | 3 |
| (20) Antiseptic | 0.1 |
| (21) Perfume | 0.05 |

| | |
|---|---|
| *21: treated with 3 parts by weight of ester oil *22: Prominence SF (Topy Industries Ltd.) *23: Xirona Caribbean Blue (Merck) *24: Xirona Magic Mauve (Merck) *25: KF-7312K (Shin-Etsu Chemical Co., Ltd.) *26: KSG-16 (Shin-Etsu Chemical Co., Ltd.) | |

### Example 19 (Foundation)

A foundation with the composition shown in Table 5 was produced.

### (Production method)

Components (1) to (8) were mixed with components (12) and (13), and the mixture was then heated to 80°C to 90°C. Components (9) to (11) were heated to 80°C to 90°C to melt them, and these components were mixed. The resulting mixture was added to the above mixture and was uniformly blended. The resulting mixture was cooled and then milled. Thereafter, the resultant product was filled in a middle plate, followed by compaction, so as to obtain a foundation.

**[Table 5]**

| (Component) | (Weight %) |
|---|---|
| (1) Talc treated with fluorine compound (average particle size: 6 µm)^{*9} | 11.45 |
| (2) Mica treated with fluorine compound (average particle size: 15 µm)^{*9} | 40 |
| (3) Sericite treated with fluorine compound (average particle size: 8 µm)^{*9} | 20 |
| (4) Silicone resin treated with fluorine compound (average particle size: 7 µm)^{*9} | 5 |
| (5) Titanium oxide treated with fluorine compound^{*9} | 10 |
| (6) Yellow iron oxide treated with fluorine compound^{*9} | 0.8 |
| (7) Black iron oxide treated with fluorine compound^{*9} | 0.1 |
| (8) Iron red treated with fluorine compound^{*9} | 2.5 |
| (9) Microcrystalline wax (penetration number: 34)^{*1} | 2 |
| (10) Dextrin palmitate^{*6} | 0.5 |
| (11) Liquid isoparaffin (viscosity: 15 mPa·s)^{*12} | 7.5 |
| (12) Antiseptic | 0.1 |
| (13) Perfume | 0.05 |

### Example 20 (solid face powder)

A solid face powder with the composition shown in Table 6 was produced.

### (Production method)

Components (1) to (8) were mixed with components (12) and (13), and the mixture was then heated to 80°C to 90°C. Components (9) to (11) were heated to 80°C to 90°C to melt them, and these components were mixed. The resulting mixture was added to the above mixture and was uniformly blended. The resulting mixture was cooled and then milled. Thereafter, the resultant product was filled in a middle plate, followed by compaction, so as to obtain a solid face powder.

**[Table 6]**

| (Component) | (Weight %) |
|---|---|
| (1) Talc treated with fluorine compound (average particle size: 6 µm)^{*9} | 35.14 |
| (2) Mica treated with fluorine compound (average particle size: 15 µm)^{*9} | 20 |
| (3) Sericite treated with fluorine compound (average particle size: 8 µm)^{*9} | 35 |
| (4) Spherical polymethyl methacrylate powder treated with fluorine compound (average particle size: 12 µm)^{*9} | 2 |
| (5) Titanium oxide treated with fluorine compound^{*9} | 0.5 |
| (6) Yellow iron oxide treated with fluorine compound^{*9} | 0.1 |
| (7) Black iron oxide treated with fluorine compound^{*9} | 0.01 |
| (8) Iron red treated with fluorine compound^{*9} | 0.1 |
| (9) Microcrystalline wax (penetration number: 34)^{*1} | 1 |
| (10) Dextrin palmitate^{*6} | 0.2 |
| (11) Liquid isoparaffin (viscosity: 15 mPa·s)^{*12} | 5.8 |
| (12) Antiseptic | 0.1 |
| (13) Perfume | 0.05 |

### Example 21 (Cheek)

Cheek with the composition shown in Table 7 was produced.

### (Production method)

Components (1) to (11) were mixed with components (15) and (16), and the mixture was then heated to 80°C to 90°C. Components (12) to (14) were heated to 80°C to 90°C to melt them, and these components were mixed. The resulting mixture was added to the above mixture and was uniformly blended. The resulting mixture was cooled and then milled. Thereafter, the resultant product was filled in a middle plate, followed by compaction, so as to obtain cheek.

**[Table 7]**

| (Component) | (Weight %) |
|---|---|
| (1) Talc treated with fluorine compound^{*9} | 28.75 |
| (2) Mica treated with fluorine compound^{*9} | 20 |
| (3) Sericite treated with fluorine compound^{*9} | 20 |
| (4) Spherical silicone resin treated with fluorine compound^{*9} | 5 |
| (5) Titanium oxide treated with fluorine compound^{*9} | 0.5 |
| (6) Yellow iron oxide treated with fluorine compound^{*9} | 0.3 |
| (7) Black iron oxide treated with fluorine compound^{*9} | 0.1 |
| (8) Blue #404 treated with fluorine compound^{*9} | 1.2 |
| (9) Mica titanium (average particle size: 20 µm) | 10 |
| (10) Mica titanium coated with iron red (average particle size: 20 µm) | 2 |
| (11) Glass powder coated with titanium oxide (average particle size: 40 µm) | 4 |
| (12) Microcrystalline wax (penetration number: 34)^{*1} | 1.6 |
| (13) Dextrin palmitate^{*6} | 0.4 |
| (14) Liquid isoparaffin (viscosity: 15 mPa·s)^{*12} | 6 |
| (15) Antiseptic | 0.1 |
| (16) Perfume | 0.05 |

### Example 22 (Eyebrow)

An eyebrow with the composition shown in Table 8 was produced.

### (Production method)

Components (1) to (8) were mixed with components (12) and (13), and the mixture was then heated to 80°C to 90°C. Components (9) to (11) were heated to 80°C to 90°C to melt them, and these components were mixed. The resulting mixture was added to the above mixture and was uniformly blended. The resulting mixture was cooled and then milled. Thereafter, the resultant product was filled in a middle plate, followed by compaction, so as to obtain an eyebrow.

**[Table 8]**

| | |
|---|---|
| (Component) | (Weight %) |
| (1) Talc treated with fluorine compound (average particle size: 6 µm)^{*9} | 29.25 |
| (2) Mica treated with fluorine compound (average particle size: 15 µm)^{*9} | 5 |
| (3) Sericite treated with fluorine compound (average particle size: 8 µm)^{*9} | 40 |
| (4) Nylon powder treated with fluorine compound (average particle size: 6 µm)^{*9} | 5 |
| (5) Titanium oxide treated with fluorine compound^{*9} | 0.5 |
| (6) Yellow iron oxide treated with fluorine compound^{*9} | 0.2 |
| (7) Black iron oxide treated with fluorine compound^{*9} | 12 |
| (8) Iron red treated with fluorine compound^{*9} | 1.5 |
| (9) Microcrystalline wax (penetration number: 34)^{*1} | 1.2 |
| (10) Dextrin palmitate^{*6} | 0.2 |
| (11) Liquid isoparaffin (viscosity: 15 mPa·s)^{*12} | 5 |
| (12) Antiseptic | 0.1 |
| (13) Perfume | 0.05 |

### Example 23 (Body powder)

A body powder with the composition shown in Table 9 was produced.

### (Production method)

Components (1) to (8) were mixed with components (12) to (14), and the mixture was then heated to 80°C to 90°C. Components (9) to (11) were heated to 80°C to 90°C to melt them, and these components were mixed. The resulting mixture was added to the above mixture and was uniformly blended. The resulting mixture was cooled and then milled. Thereafter, the resultant product was filled in a middle plate, followed by compaction, so as to obtain a body powder.

**[Table 9]**

| (Component) | (Weight %) |
|---|---|
| (1) Talc treated with fluorine compound^{*9} | 26.14 |
| (2) Mica treated with fluorine compound^{*9} | 30 |
| (3) Sericite treated with fluorine compound^{*9} | 30 |
| (4) Spherical silicone resin treated with fluorine compound^{*9} | 5 |
| (5) Titanium oxide treated with fluorine compound^{*9} | 0.5 |
| (6) Yellow iron oxide treated with fluorine compound^{*9} | 0.1 |
| (7) Black iron oxide treated with fluorine compound^{*9} | 0.01 |
| (8) Iron red treated with fluorine compound^{*9} | 0.1 |
| (9) Microcrystalline wax (penetration number: 34)^{*1} | 1.5 |
| (10) Dextrin palmitate^{*6} | 0.4 |
| (11) Liquid isoparaffin (viscosity:15 mPa·s)^{*12} | 6 |
| (12) Antiphlogistic | 0.1 |
| (13) Antiseptic | 0.1 |
| (14) Perfume | 0.05 |

All the cosmetic pressed powder obtained in Examples 17 to 23 were excellent in terms of fine attachment of the cosmetics to an applicator, spreadability of the cosmetics when it was applied, conformability, a finely textured finish, a long-lasting property, and impact resistance.

## Claims

1. A cosmetic pressed powder **characterized by** comprising a wax (A), a dextrin fatty acid ester (B), a liquid oil (C), and a powder (D), wherein the content of the component (D) is 80% by weight or more.

2. The cosmetic pressed powder according to claim 1, **characterized in that** the component (A) includes a wax having a penetration number of between 20 and 110 at 25°C.

3. The cosmetic pressed powder according to claim 1 or 2, **characterized in that** it comprises, as a powder (D), 20 % by weight or more of a pearlescent pigment having an average particle size of 10 µm or greater based on the total composition.

4. The cosmetic pressed powder according to any one of claims 1 to 3, **characterized in that** the weight ratio (B)/(A) between the component (A) and the component (B) is between 0.1 and 0.5.

5. The cosmetic pressed powder according to any one of claims 1 to 4, **characterized in that** the weight ratio ((A)+(B))/(C) of the total weight of the component (A) and the component (B) and the component (C) is between 0.14 and 1.1.

6. A method for producing a cosmetic pressed powder, **characterized in that** the method comprises heating and dissolving a wax (A) and a dextrin fatty acid ester (B) in a liquid oil (C), then adding a powder (D) thereto, and compacting the obtained mixture, wherein the content of the powder (D) is 80% by weight or more.

## Patentansprüche

1. Kosmetisches komprimiertes Pulver, **dadurch gekennzeichnet, dass** es ein Wachs (A), einen Dextrinfettsäureester (B), ein flüssiges Öl (C) und ein Pulver (D) umfasst, worin der Gehalt der Komponente (D) 80 Gew.% oder mehr ist.

2. Kosmetisches komprimiertes Pulver nach Anspruch 1, **dadurch gekennzeichnet, dass** die Komponente (A) ein Wachs mit einer Penetrationszahl zwischen 20 und 110 bei 25°C umfasst.

3. Kosmetisches komprimiertes Pulver nach Anspruch 1 oder 2, **dadurch gekennzeichnet, dass** es als Pulver (D) 20 Gew.% oder mehr eines Perlpigmentes mit einer durchschnittlichen Teilchengröße von 10 µm oder mehr bezogen auf die gesamte Zusammensetzung umfasst.

4. Kosmetisches komprimiertes Pulver nach einem der Ansprüche 1 bis 3, **dadurch gekennzeichnet, dass** das Gewichtsverhältnis (B)/(A) zwischen der Komponente (A) und der Komponente (B) zwischen 0,1 und 0,5 ist.

5. Kosmetisches komprimiertes Pulver nach einem der Ansprüche 1 bis 4, **dadurch gekennzeichnet, dass** das Gewichtsverhältnis ((A)+(B))/(C) des Gesamtgewichtes der Komponente (A) und der Komponente (B) und der Komponente (C) zwischen 0,14 und 1,1 liegt.

6. Verfahren zur Erzeugung eines kosmetischen komprimierten Pulvers **dadurch gekennzeichnet, dass** das Verfahren das Erwärmen und Auflösen eines Wachses (A) und eines Dextrinfettsäureesters (B) in einem flüssigen Öl (C), die anschließende Zugabe eines Pulvers (D) und das Kompaktieren der erhaltenen Mischung umfasst, worin der Gehalt des Pulvers (D) 80 Gew.% oder mehr ist.

## Revendications

1. Poudre cosmétique compacte **caractérisée en ce qu'**elle comprend une cire (A), un ester d'acide gras de dextrine (B), une huile liquide (C), et une poudre (D), dans laquelle la teneur en le composant (D) est de 80 % en poids ou plus.

2. Poudre cosmétique compacte selon la revendication 1, **caractérisée en ce que** le composant (A) inclut une cire possédant un indice de pénétration situé entre 20 et 110 à 25 °C.

3. Poudre cosmétique compacte selon la revendication 1 ou 2, **caractérisée en ce qu'**elle comprend, en tant que poudre (D), 20 % en poids ou plus d'un pigment nacré possédant une taille de particule moyenne de 10 µm ou plus sur la base de la composition totale.

4. Poudre cosmétique compacte selon l'une quelconque des revendications 1 à 3, **caractérisée en ce que** le rapport pondéral (B)/(A) entre le composant (A) et le composant (B) se situe entre 0,1 et 0,5.

5. Poudre cosmétique compacte selon l'une quelconque des revendications 1 à 4, **caractérisée en ce que** le rapport pondéral ((A) + (B)/(C) du poids total du composant (A) et du composant (B) et le composant (C) se situe entre 0,14 et 1,1.

6. Procédé destiné à produire une poudre cosmétique compacte, **caractérisé en ce que** le procédé comprend de chauffer et de dissoudre une cire (A) et un ester d'acide gras de dextrine (B) dans une huile liquide (C), puis d'ajouter une poudre (D) à ceci, et de compacter le mélange obtenu, dans lequel la teneur en la poudre (D) est de 80 % ou plus.
